# EUROPEAN PATENT APPLICATION

(11) **EP 2 253 270 A1**
(43) Date of publication of application: **24.11.2010**
(21) Application number: 09722512.2
(22) Date of filing: 21.01.2009
(51) Int. Cl.: A61B 5/157, A61B 5/1473, A61B 5/151

(54) **CIRCUIT BOARD FOR COLLECTING BODY FLUID, METHOD OF MANUFACTURING THE BOARD, METHOD FOR USE OF THE BOARD, AND BIOSENSOR**

(30) Priority: 21.03.2008 JP 2008073542
(71) Applicant: Nitto Denko Corporation, Ibaraki-shi Osaka 567-8680 (JP)
(72) Inventor: NAITO, Toshiki, Ibaraki-shi Osaka 567-8680 (JP); ISHIGAKI, Saori, Ibaraki-shi Osaka 567-8680 (JP); OHSAWA, Tetsuya, Ibaraki-shi Osaka 567-8680 (JP)
(74) Representative: HOFFMANN EITLE
(86) International application number: PCT/JP2009/050830
(87) International publication number: WO 2009/116314

(57) **Abstract**

A circuit board for body fluid collection includes a plurality of measurement units including a puncture needle and an electrode for making contact with a body fluid collected by puncturing with the puncture needle, the plurality of measurement units being arranged in parallel in a predetermined direction, and a support portion extending along the parallel arrangement direction and supporting the plurality of measurement units, wherein the support portion is rolled so that the plurality of measurement units can be disposed radially.

## Description

### TECHNICAL FIELD

The present invention relates to a circuit board for body fluid collection, a method for producing the circuit board for body fluid collection, a method for using the circuit board for body fluid collection, and a biosensor including the circuit board for body fluid collection.

### BACKGROUND ART

Diabetes mellitus includes insulin-dependent (type I) diabetes and non-insulin-dependent (type II) diabetes. The former type of diabetes necessitates regular administration of insulin. Therefore, for a patient with the former type of diabetes, there has been employed a treatment method in which a patient collects his or her blood, measures his or her blood sugar level, and administers to himself or herself insulin at a dosage in accordance with the blood sugar level.
There has been known, for mainly such patients, a blood-sugar-level measuring device which allows a patient to personally collect blood on his/her own and to measure a blood sugar level.

For example, there has been proposed a fluid collecting device including a reaction zone which is provided at the center of a main body and into which electrodes are inserted; a puncture needle outwardly protruding from the center of the main body; and a capillary channel providing communication between the electrodes and the puncture needle (ref: for example, Patent Document 1 shown below).
[Patent Document 1]
Japanese Unexamined Patent Publication No. 2004-493

### DISCLOSURE OF THE INVENTION

### PROBLEM TO BE SOLVED BY THE INVENTION

In the above-described fluid collecting device of Patent Document 1, the puncture needle and the reaction zone are formed integrally with the main body, and therefore the measurement preparation is easy. However, with this fluid collecting device, the electrode which is a member separate from the reaction zone has to be inserted into the reaction zone to perform a measurement on the blood component. Therefore, there is a disadvantage in that blood detection accuracy becomes unstable and accurate measurement cannot be performed.
Moreover, in type I diabetes, depending on symptoms, the patient has to measure the blood-sugar level several times per day, to be specific, before every meal or after every meal.

However, with the above-described fluid collecting device of Patent Document 1, only one puncture needle is provided in one device, and therefore in order to avoid repetitive use of the puncture needle, the measurement can be performed only once.
Therefore, when the measurement is performed several times as described above with the above-described fluid collecting device, it is necessary that the used fluid collecting device is disposed and a new fluid collecting device is prepared afterwards. Thus, with such a fluid collecting device, the measurement preparation as described above is complicated, and an increase in running costs is inevitable.

An object of the present invention is to provide a circuit board for body fluid collection that allows accurate measurement on a body fluid component with a simple structure, and even allows easy measurement a plurality of times with one circuit board for body fluid collection; a method for producing the same; a method for using the same; and a biosensor including the circuit board for body fluid collection.

### MEANS FOR SOLVING THE PROBLEM

To achieve the above-described object, a circuit board for body fluid collection of the present invention includes a plurality of measurement units including a puncture needle and an electrode for making contact with a body fluid collected by puncturing with the puncture needle, the plurality of measurement units being arranged in parallel in a predetermined direction; and a support portion extending along the parallel arrangement direction and supporting the plurality of measurement units, wherein the support portion can be rolled so that the plurality of measurement units are arranged radially.

The circuit board for body fluid collection includes the measurement unit including the puncture needle and the electrode. Thus, by causing a body fluid to flow out by puncturing with the puncture needle, the body fluid that was caused to flow out is easily brought into contact with the electrode in the measurement unit. As a result, with the circuit board for body fluid collection, a measurement on a component in body fluid can be performed easily with a simple structure.
Furthermore, with the circuit board for body fluid collection, by using the plurality of measurement units provided in one circuit board for body fluid collection, a measurement on a component in the body fluid can be performed a plurality of times.

Moreover, with the circuit board for body fluid collection, by rolling the support portion and thereby arranging the plurality of measurement units radially, after one measurement unit is used, the used measurement unit can be changed to an unused measurement unit that is at an upstream side of and adjacent to the used measurement unit in the rotational direction, by rotating the circuit board for body fluid collection in a circumferential direction. Therefore, at every measurement in a plurality of measurements, the measurement unit can be changed easily.

It is preferable that, in the circuit board for body fluid collection of the present invention, the plurality of measurement units extend outwardly with respect to the support portion when the plurality of measurement units are arranged radially.
With the circuit board for body fluid collection, the plurality of measurement units allow reliable puncturing and measurement at an outside of the support portion that was rolled.

It is preferable that, in the circuit board for body fluid collection of the present invention, the plurality of measurement units extend inwardly with respect to the support portion when the plurality of measurement units are arranged radially.
With the circuit board for body fluid collection, the plurality of measurement units allow reliable puncturing and measurement at an inside of the support portion that was rolled.

It is preferable that the circuit board for body fluid collection of the present invention further includes a reinforcing portion that is disposed in a spaced apart relationship to the support portion, and connects, between the plurality of measurement units, the plurality of measurement units that are adjacent to each other.
With the circuit board for body fluid collection, even though the plurality of measurement units are arranged radially, because the plurality of measurement units are connected with the reinforcing portion, the plurality of measurement units can be reliably supported. Therefore, reliable puncturing and measurement by the measurement unit can be achieved.

It is preferable that the circuit board for body fluid collection of the present invention further includes engage portions at one end portion and at the other end portion of the support portion in the parallel arrangement direction for retaining the rolling of the support portion.
With the circuit board for body fluid collection, when the support portion is rolled, the rolling of the support portion is retained by engaging the engage portion at the one end portion of the support portion with the engage portion at the other end portion of the support portion in the parallel arrangement direction. Thus, the plurality of measurement units can be reliably arranged radially.

A circuit board for body fluid collection includes: a plurality of measurement units including a puncture needle and an electrode for making contact with a body fluid collected by puncturing with the puncture needle, the plurality of measurement units being arranged in parallel in a predetermined direction; and a support portion extending along the parallel arrangement direction and supporting the plurality of measurement units, wherein the plurality of measurement units are arranged radially by bending a boundary between the plurality of measurement units and the support portion, and rolling the support portion.

The circuit board for body fluid collection includes the measurement unit including the puncture needle and the electrode. Thus, by causing a body fluid to flow out by puncturing with the puncture needle, the body fluid that was caused to flow out is easily brought into contact with the electrode in the measurement unit. As a result, with the circuit board for body fluid collection, a measurement on a component in body fluid can be performed easily with a simple structure.
Furthermore, with the circuit board for body fluid collection, by using the plurality of measurement units provided in one circuit board for body fluid collection, a measurement on a component in the body fluid can be performed a plurality of times.

Moreover, with the circuit board for body fluid collection, by rolling the support portion and thereby arranging the plurality of measurement units radially, after one measurement unit is used, the used measurement unit can be changed to an unused measurement unit that is at an upstream side of and adjacent to the used measurement unit in the rotational direction, by rotating the circuit board for body fluid collection in a circumferential direction. Therefore, at every measurement in a plurality of measurements, the measurement unit can be changed easily.

It is preferable that, in the circuit board for body fluid collection of the present invention, the plurality of measurement units extend outwardly with respect to the support portion.
With the circuit board for body fluid collection, the plurality of measurement units allow reliable puncturing and measurement at an outside of the support portion that was rolled.
It is preferable that, in the circuit board for body fluid collection of the present invention, the plurality of measurement units extend inwardly with respect to the support portion.

With the circuit board for body fluid collection, the plurality of measurement units allow reliable puncturing and measurement at an inside of the support portion that was rolled.
Furthermore, a biosensor of the present invention includes the above-described circuit board for body fluid collection, and a determination unit that is electrically connected to the electrodes and performs a measurement on a component of the body fluid.
With the biosensor, a measurement on a component in body fluid can be easily performed in such a way that the body fluid that was caused to flow out by the above-described circuit board for body fluid collection is brought into contact with the electrode, and then a measurement is performed with the determination unit that is electrically connected to the electrode.

Furthermore, a method for producing a circuit board for body fluid collection of the present invention includes the steps of: preparing a metal substrate; forming an insulating layer on the metal substrate; forming an electrode for making contact with a body fluid on the insulating layer; and forming, by trimming the metal substrate, a plurality of measurement units including a puncture needle for collecting a body fluid by puncturing and the electrode, the plurality of measurement units being arranged in parallel in a predetermined direction, and a support portion extending along the parallel arrangement direction and supporting the plurality of measurement units, wherein in the step of forming the plurality of measurement units and the support portion, the metal substrate is trimmed so that by rolling the support portion, the plurality of measurement units can be arranged radially.

With the method for producing a circuit board for body fluid collection, by rolling the support portion, the plurality of measurement units can be arranged radially. Thus, by arranging the circuit board for body fluid collection in such a manner, after one measurement unit is used, the used measurement unit can be changed to an unused measurement unit that is at an upstream side of and an adjacent to the used measurement unit in the rotational direction, by rotating the circuit board for body fluid collection in a circumferential direction. Therefore, at every measurement in a plurality of measurements, the measurement unit can be changed easily.

Moreover, with the method for producing a circuit board for body fluid collection, by trimming the metal substrate, the plurality of measurement units and the support portion are arranged in parallel in a predetermined direction. Therefore, yields of the circuit board for body fluid collection can be improved, and an improvement in production efficiency allows reduction in costs.
Furthermore, a method for using a circuit board for body fluid collection of the present invention, in which the above-described circuit board for body fluid collection produced by the method for producing a circuit board for body fluid collection is used, includes the steps of: bending a boundary between the plurality of measurement units and the support portion, and rolling the support portion, thereby arranging the plurality of measurement units radially.

With the method for using a circuit board for body fluid collection, by bending a boundary between the plurality of measurement units and the support portion, and rolling the support portion that was bent, the plurality of measurement units can be arranged radially. Therefore, the plurality of measurement units can be arranged radially by easy procedures.

### EFFECTS OF THE INVENTION

With the circuit board for body fluid collection according to the present invention, a measurement on a component in body fluid can be performed a plurality of times with the measurement unit that is provided in a plural number in one circuit board for body fluid collection while an easy measurement on a component in body fluid is achieved with a simple structure. Furthermore, at every measurement in a plurality of measurements, the measurement unit can be easily changed.
Furthermore, with the biosensor according to the present invention, a measurement on a component in body fluid can be easily performed.

Furthermore, with the method for producing the circuit board for body fluid collection according to the present invention, the measurement unit can be changed easily at every measurement in a plurality of measurements, while costs are reduced by improving yields of the circuit board for body fluid collection.
Furthermore, with the method for using the circuit board for body fluid collection, a plurality of measurement units can be arranged radially by easy procedures.

### BRIEF DESCRIPTION OF THE DRAWINGS

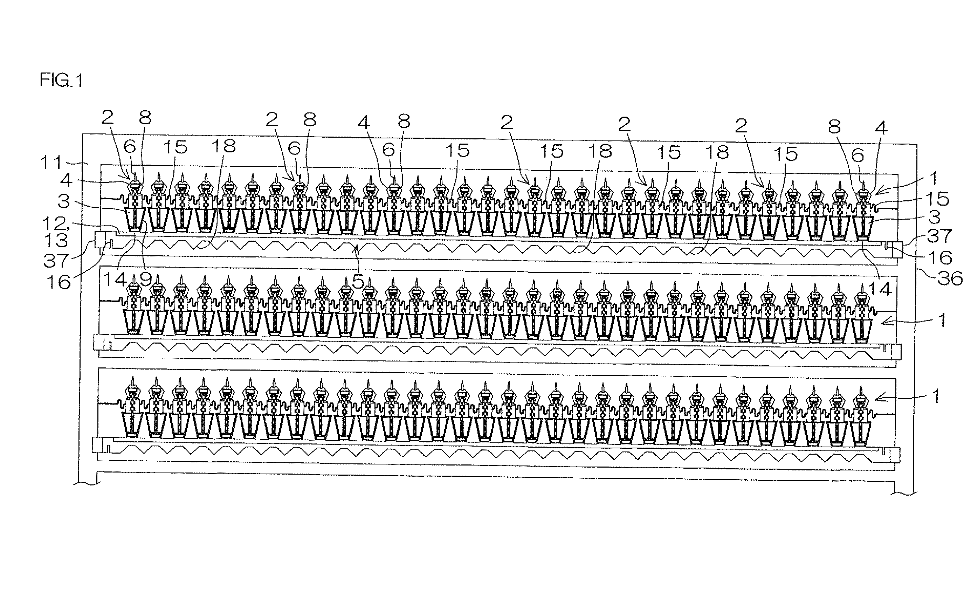

[FIG. 1] FIG. 1 shows a plan view of a circuit board for blood collection in an embodiment of the circuit board for body fluid collection of the present invention.

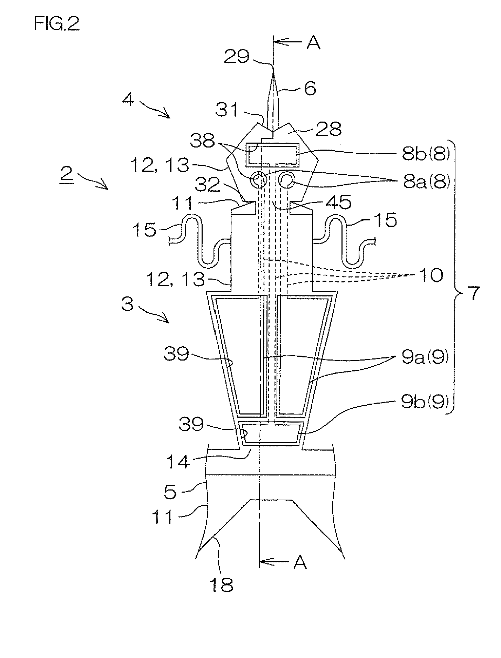

[FIG. 2] FIG. 2 shows an enlarged plan view of a measurement unit of the circuit board for blood collection shown in FIG. 1.

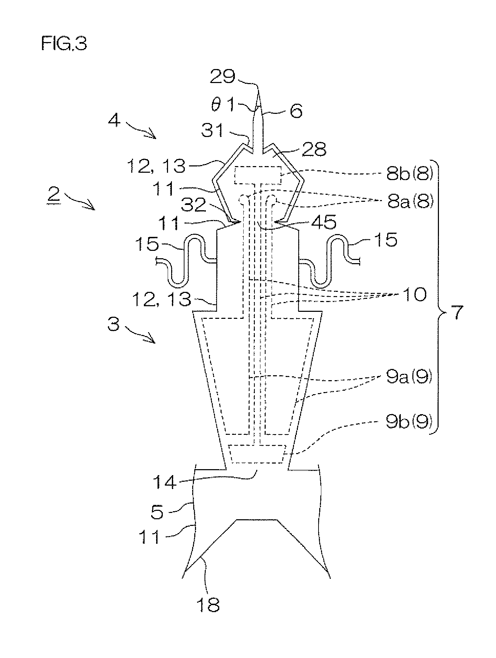

[FIG. 3] FIG. 3 shows an enlarged rear view of a measurement unit of the circuit board for blood collection shown in FIG. 1.

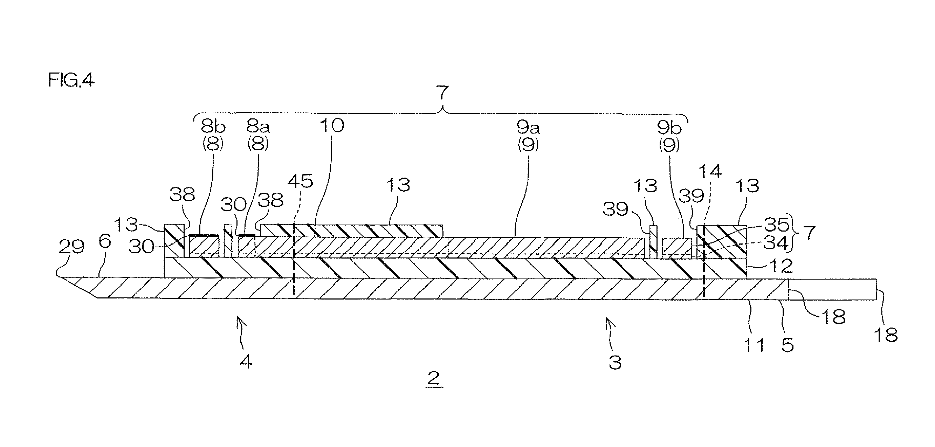

[FIG. 4] FIG. 4 shows a cross-sectional view taken along line A-A in FIG. 2.

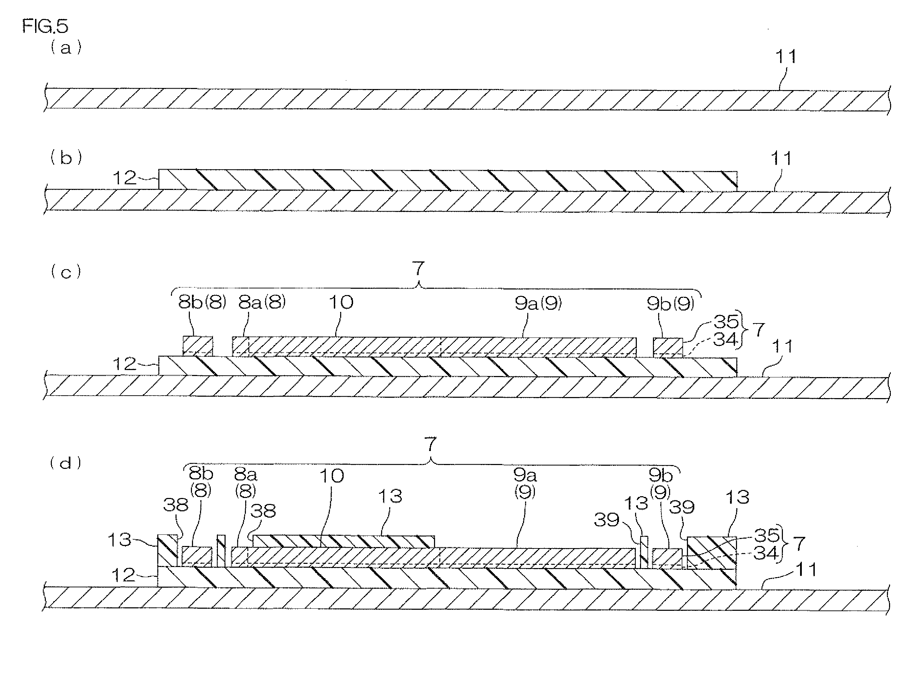

[FIG. 5] FIG. 5 is a process drawing for describing an embodiment of a method for producing a circuit board for collection of the present invention: (a) illustrating a step of preparing a metal substrate; (b) illustrating a step of forming an insulating base layer; (c) illustrating a step of forming a conductive pattern including three electrodes; and (d) illustrating a step of forming an insulating cover layer.

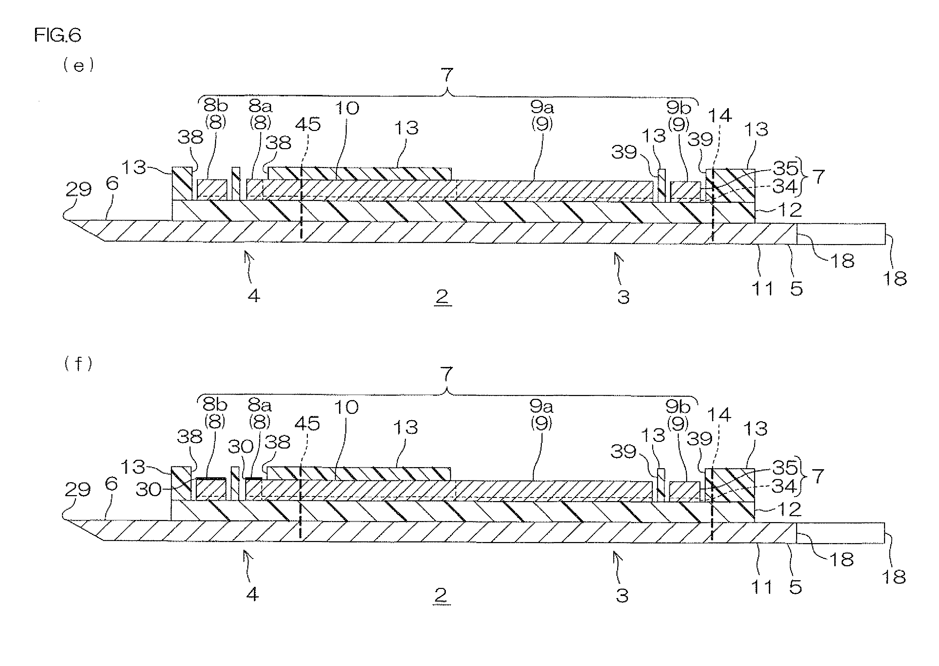

[FIG. 6] FIG. 6 is a process drawing for describing a method for producing a circuit board for collection subsequent to FIG. 5: (e) illustrating a step of trimming a metal substrate to form a plurality of measurement units including a puncture needle, and a support portion; and (f) illustrating a step of applying a chemical agent to the electrodes.

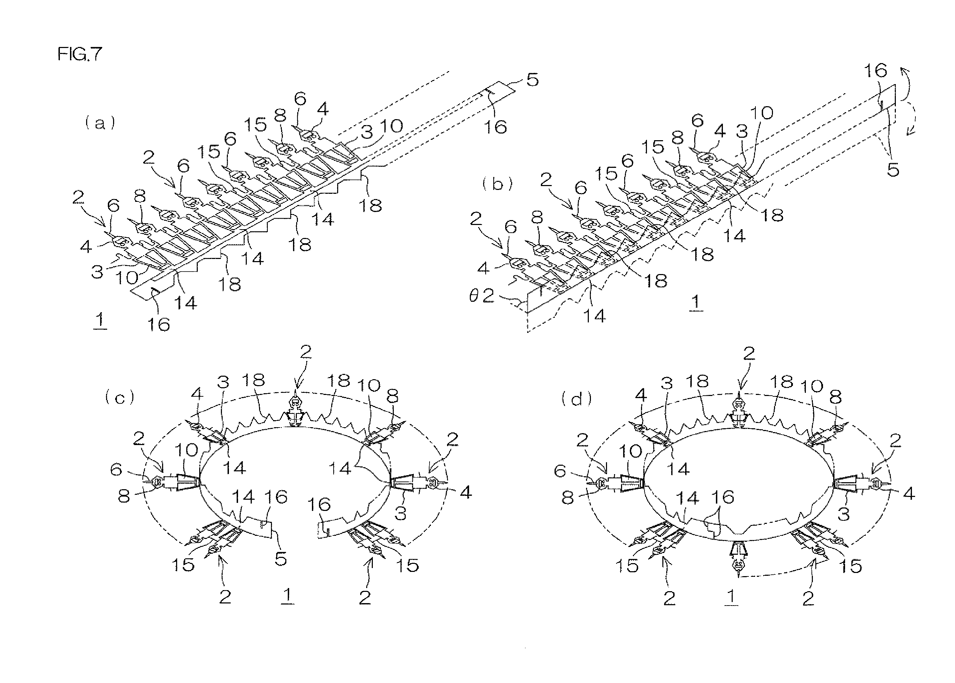

[FIG. 7] FIG. 7 shows schematic perspective views for describing an embodiment of a method for using a circuit board for body fluid collection of the present invention: (a) illustrating a step of preparing a circuit board for blood collection; (b) illustrating a step of bending a support portion at a second bending portion with respect to a plurality of measurement units; (c) illustrating a step in the middle of rolling the support portion; and (d) illustrating a step of rolling the support portion to arrange the plurality of measurement units radially.

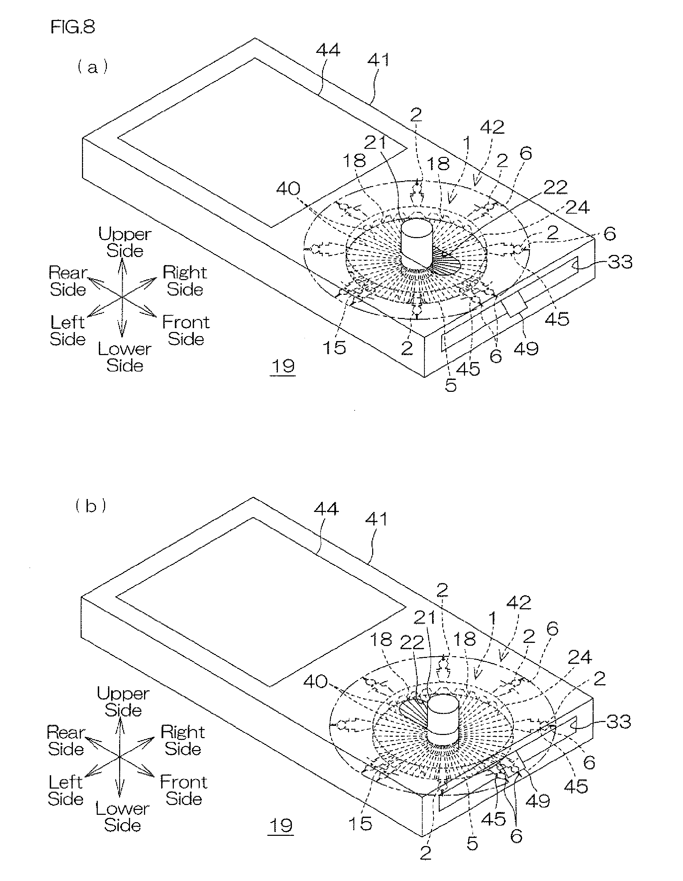

[FIG. 8] FIG. 8 shows schematic perspective views of a blood-sugar-level measuring device in which the circuit board for blood collection shown in FIG. 7 is mounted as an embodiment of a biosensor of the present invention: (a) illustrating a step of preparing a blood-sugar-level measuring device in which a driving shaft is disposed at a rear side; and (b) illustrating a step of exposing the puncture needle from a front side opening by sliding the driving shaft toward a front side.

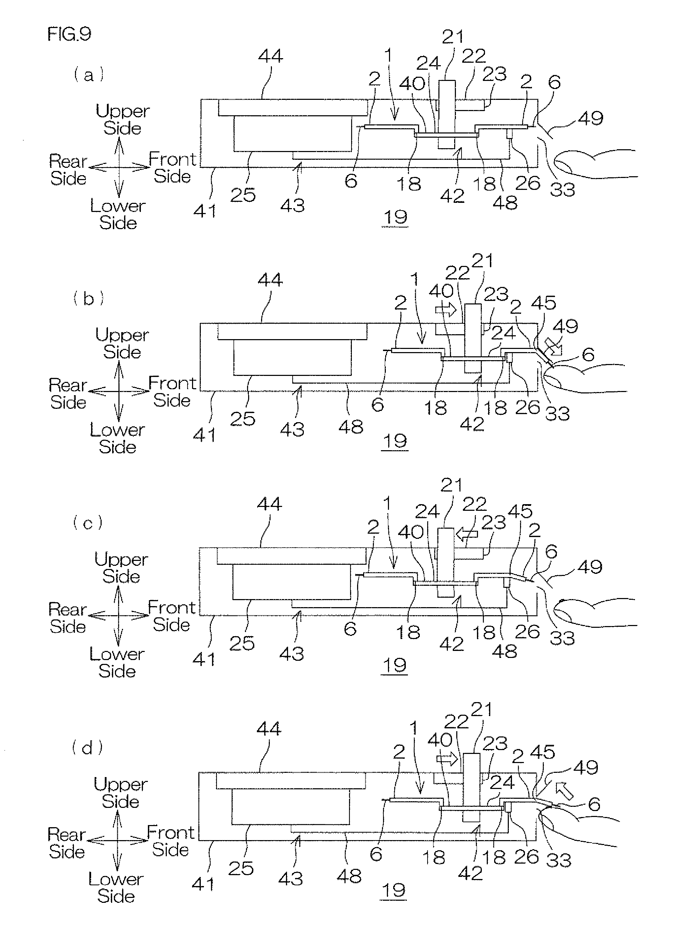

[FIG. 9] FIG. 9 shows side sectional views for describing a method for using the blood-sugar-level measuring device shown in FIG. 8: (a) illustrating a step of preparing a blood-sugar-level measuring device in which a driving shaft is disposed at the rear side; (b) illustrating a step of sliding the driving shaft toward the front side and puncturing a finger with the puncture needle; (c) illustrating a step of sliding the driving shaft to the rear side and pulling out the puncture needle to cause a trace amount of bleeding; and (d) illustrating a step of bringing the electrodes close to and into contact with the punctured portion.

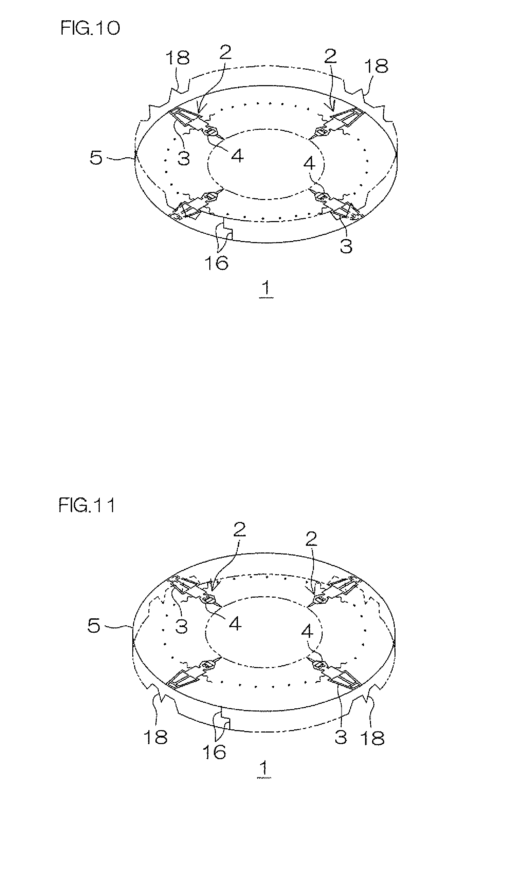

[FIG. 10] FIG. 10 shows a schematic perspective view for describing another embodiment (embodiment in which the measurement units extend inwardly in the radial direction with respect to the support portion) of a method for using a circuit board for body fluid collection of the present invention (embodiment in which the support portion is bent upward with respect to the plurality of measurement units).
[FIG. 11] FIG. 11 shows a schematic perspective view for describing another embodiment (embodiment in which the measurement units extend inwardly in the radial direction with respect to the support portion) of the method for using a circuit board for body fluid collection of the present invention (embodiment in which the support portion is bent downward with respect to the plurality of measurement units).

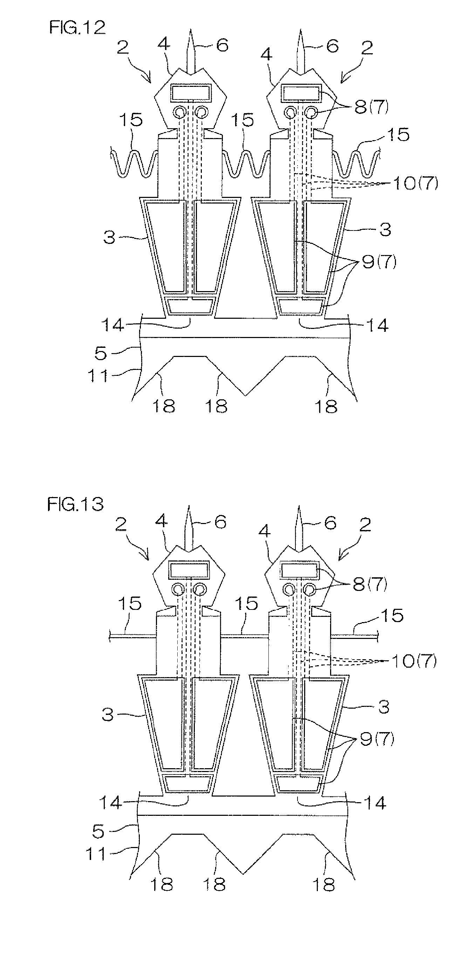

[FIG. 12] FIG. 12 shows an enlarged plan view of measurement units (embodiment in which a connector portion is generally W-shaped when viewed from top) of a circuit board for blood collection as another embodiment of the circuit board for body fluid collection of the present invention.
[FIG. 13] FIG. 13 shows an enlarged plan view of measurement units (embodiment in which the connector portion is a straight line when viewed from top) of a circuit board for blood collection of another embodiment of the circuit board for body fluid collection of the present invention.

### EMBODIMENT OF THE INVENTION

FIG. 1 shows a plan view of a circuit board for blood collection of an embodiment of the circuit board for body fluid collection of the present invention; FIG. 2 shows an enlarged plan view of a measurement unit of the circuit board for blood collection shown in FIG. 1; FIG. 3 shows an enlarged rear view of a measurement unit of the circuit board for blood collection shown in FIG. 1; FIG. 4 shows a cross-sectional view taken along line A-A of FIG. 2; and FIGs. 5 and 6 are process drawings for describing an embodiment of a method for producing a circuit board for collection of the present invention.

A circuit board for blood collection 1 shown in FIG. 1 is mounted and then used in a blood-sugar-level measuring device 19 (ref: FIGs. 8 and 9) as a biosensor to be mentioned later, for a patient to puncture his/her skin of, for example, finger to collect blood, to measure a glucose level in the collected blood. The circuit board for blood collection 1 is prepared as a multiple use (consecutively usable) type, which enables a plurality of measurements.
A plurality of the circuit boards for blood collection 1 are arranged, for example, in parallel in a frame portion 36 of an elongated sheet extending in the longitudinal direction (up and down directions in FIG. 1, corresponding to the puncture direction to be mentioned later), along the longitudinal direction of the frame portion 36. To be specific, the circuit boards for blood collection 1 are arranged in an orderly manner in the longitudinal direction with a space provided therebetween. The circuit boards for blood collection 1 are supported by the frame portion 36 through a joint portion 37, and are formed so as to extend in the direction (in the following, referred to as width direction) perpendicular to the longitudinal direction.

The joint portions 37 are extended from widthwise both end portions of the frame portion 36 to widthwise both end portions of the circuit board for blood collection 1 so as to be removable therefrom.
The circuit board for blood collection 1 integrally includes a support portion 5 extending along the width direction, and a plurality (32 units) of measurement units 2 that are supported by the support portion 5 and extending along the longitudinal direction.
The support portion 5 is formed into a generally belt shape extending in the width direction when viewed from top, and has a gear 18, and a slit portion 16 as an engage portion formed therein.

The gear 18 is formed at a substantially entire face of the other end face in the longitudinal direction (end face at the upstream side in the puncture direction) of the support portion 5 (except for widthwise both end portions) so that the gear 18 can be engaged with a gear plate 24 (FIG. 9) to be mentioned later. To be specific, the gear 18 is formed into a sawtooth shape with projections and depressions alternately arranged.
The slit portions 16 are provided at widthwise both end portions of the support portion 5. To be specific, the slit portion 16 at a widthwise one end portion (in FIG. 1, left end portion) of the support portion 5 is formed like a slit cutting in from the longitudinal other end face of the support portion 5 toward a longitudinal one side up to a halfway in the longitudinal direction. The slit portion 16 at a widthwise other end portion (right end portion in FIG. 1) is formed like a slit cutting in from the longitudinal one end face of the support portion 5 toward a longitudinal other side of the support portion 5 up to a halfway in the longitudinal direction. In this way, the slit portions 16 at widthwise both end portions engage with each other when rolling the support portion 5, which is to be mentioned later.

The measurement unit 2 is disposed, as shown in FIGs. 2 and 3, so as to protrude toward a longitudinal direction one side (downstream side in the puncture direction, upper side in FIGs. 2 and 3) from the support portion 5. The measurement unit 2 integrally includes an upstream-side portion 3 disposed at an upstream side in the puncture direction, and a downstream-side portion 4 disposed at a downstream side in the puncture direction of the upstream-side portion 3.
As shown in FIG. 1, the upstream-side portion 3 is formed so that the upstream-side portion 3 is disposed continuously in the puncture direction with the downstream side end portion in the puncture direction of the support portion 5 to be adjacent with each other, and disposed to face the upstream-side portion 3 of the measurement unit 2 that is adjacent in the width direction with a space provided therebetween in the width direction. The upstream-side portion 3 integrally includes a base portion in which a terminal 9 (described later) is provided, having a generally trapezoid shape when viewed from top, with its width gradually increasing toward a downstream side in the puncture direction; and a middle portion to which a connector portion 15 (described later) is connected, protruding from the base portion toward the downstream side in the puncture direction and having generally a rectangular shape when viewed from top.

The downstream side end face in the puncture direction of the upstream-side portion 3 is formed, as shown in FIGs. 2 and 3, into a generally arc shape (or generally straight) when viewed from top, and in this way, as shown in FIG. 7(d), the circuit board for blood collection 1 is formed into a generally disc shape with intermittently continuous arc when the support portion 5 is rolled. The length of the upstream-side portion 3 in the width direction (that is, the length of the measurement unit 2 in the width direction) is, for example, 1 to 5 mm.

The downstream-side portion 4 is disposed, as shown in FIGs. 2 and 3, so as to protrude from a generally center in the width direction of the downstream side end face in the puncture direction of the upstream-side portion 3 toward the downstream side in the puncture direction. The downstream-side portion 4 is formed so that the length thereof in the width direction is shorter than that of the upstream-side portion 3. The downstream-side portion 4 is formed from an electrode portion 28 that is formed into a generally regular pentagon when viewed from top; and a puncture needle 6 that is disposed at a downstream side in the puncture direction of the electrode portion 28.

The measurement unit 2 includes one puncture needle 6 and a conductive pattern 7.
The puncture needle 6 is provided to collect blood by puncturing. That is, the puncture needle 6 is disposed, in the downstream-side portion 4, adjacent to the electrode portion 28 at a downstream side in the puncture direction, and formed integrally with the electrode portion 28. To be specific, the puncture needle 6 protrudes from a center in the width direction of the downstream side end portion in the puncture direction of the electrode portion 28 toward the downstream side in the puncture direction. The puncture needle 6 is formed into a generally triangle shape (isosceles triangle) with its distal end 29 (downstream side end portion in the puncture direction) tapered along the puncture direction to form an acute angle when viewed from top.

Angle θ1 (ref: FIG. 3) of the distal end 29 of the puncture needle 6 is, for example, 10 to 30°, or preferably 15 to 25°. When the angle θ1 of the distal end 29 is below 10°, the skin puncturing may not be performed because of insufficient strength. On the other hand, when the angle θ1 exceeds 30°, the puncturing may become difficult. The length of the puncture needle 6 in the puncture direction is, for example, 0.5 to 10 mm, and the length of the puncture needle 6 in the width direction (width of the upstream-side portion in the puncture direction) is, for example, 0.1 to 3 mm. When the length in the puncturing direction and the width of the puncture needle 6 are below the above-described range, the blood collection may become difficult, and when the length in the puncturing direction and the width of the puncture needle 6 exceed the above-described range, damage at the punctured portion may increase.

The conductive pattern 7 includes three electrodes 8, three terminals 9, and three wirings 10.
The three electrodes 8 are provided to be brought into contact with blood that is collected by the puncturing with the puncture needle 6, and are disposed adjacently in the width direction and in the puncture direction in the electrode portion 28.
To be more specific, two electrodes 8a among the three electrodes 8 are disposed to face each other with a space provided therebetween in the width direction in the electrode portion 28. The two electrodes 8a are formed into a generally circular shape when viewed from top.

The remaining one electrode 8b is disposed in the electrode portion 28 at a downstream side in the puncture direction to face the two electrodes 8b with a space provided therebetween. The electrode 8b is formed into a generally rectangular shape, and extends over the two electrodes 8b in the width direction when viewed from top.
The three electrodes 8 correspond to a working electrode, a counter electrode, and a reference electrode, respectively. The diameter of the two electrodes 8a is, for example, 100 µm to 5 mm, and the length of a side of the one electrode 8b is, for example, 100 µm to 2.5 mm. The three electrodes 8 are disposed, for example, within 0.2 to 5 mm, or preferably 0.5 to 3 mm of the distal end 29 of the puncture needle 6 in the puncture direction. When the space between the distal end 29 of the puncture needle 6 and the electrodes 8 is too short, the electrodes 8 sting the skin along with the puncture needle 6, and a chemical agent 30 (described later) applied to the surface of the electrodes 8 may be dispersed into the body, which may hinder accurate measurements. On the other hand, when the space between the distal end 29 of the puncture needle 6 and the electrodes 8 is too long, a structure for utilizing aspiration or capillarity to introduce blood from the puncture needle 6 to the electrodes 8 becomes necessary.

The three terminals 9 are provided in correspondence with the three electrodes 8, and are disposed at the base portion of the upstream-side portion 3 to be connected to a CPU 25 to be mentioned later.
To be more specific, two terminals 9a correspond to the two electrodes 8a, and are disposed to face each other with a space provided therebetween in the width direction in the base portion of the upstream-side portion 3. The remaining one terminal 9b corresponds to the one electrode 8b, and is disposed at an upstream side in the puncture direction with respect to the two terminals 9a to face thereto with a space provided therebetween.

The three terminals 9 are formed into a generally tapered shape when viewed from top, and the width thereof gradually widens toward a downstream side in the puncture direction. To be specific, the widthwise internal end edges of the two terminals 9a facing each other are arranged in parallel along the puncture direction. The widthwise external end edge of the two terminals 9a, and widthwise both end edges of the remaining one terminal 9b are formed along directions that cross the puncture direction.

The length of a side of the three terminals 9 is, for example, 0.5 to 5 mm.
The three wirings 10 are provided so as to run through the upstream-side portion 3 and the downstream-side portion 4, and are arranged in parallel with a space provided therebetween in the width direction. The three wirings 10 are provided along the puncture direction so as to electrically connect respective electrodes 8 and the terminals 9 corresponding to the electrodes 8. The respective electrodes 8, respective terminals 9, and the wirings 10 that allow connection between them are provided continuously and integrally. The length of the wirings 10 in the width direction is, for example, 0.01 to 2 mm, and the length of the wirings 10 in the puncture direction is, for example, 2 to 28 mm.

The measurement unit 2 has a connector portion 15 as a reinforcing portion, a first bending portion 45, and a stopper portion 31.
The connector portion 15 is disposed at a downstream side in the puncture direction in a spaced apart relationship to the support portion 5, and allows connection between the downstream-side portions 4 of the measurement units 2 that are adjacent to each other. To be specific, the connector portion 15 is extended between the middle portions of the downstream-side portions 4 so as to wind like a generally S-shape when viewed from top. The connector portion 15 is formed, as shown in FIG. 7(d), so that the winding portion is extended to a generally straight line when viewed from top when the support portion 5 is rolled.

The first bending portion 45 is provided, as shown in FIG. 9, so as to be bendable at an upstream side in the puncture direction with respect to the distal end 29 of the puncture needle 6. That is, the first bending portion 45 is formed, as shown in FIGs. 2 and 3, as a straight line portion extending along the width direction between the upstream-side portion 3 and the downstream-side portion 4.
The first bending portion 45 is formed at an adjacent portion where the upstream-side portion 3 and the downstream-side portion 4 are adjacent to each other, by a cutting portion 32 that is cut finely toward an inner side in the width direction, as an hourglass portion that is narrow in width.

In this fashion, the first bending portion 45 is formed as a fragile portion between the upstream-side portion 3 and the downstream-side portion 4, and therefore the first bending portion 45 is provided so that the downstream-side portion 4 is bendable with respect to the upstream-side portion 3.
The stopper portion 31 is provided, in the downstream-side portion 4, at a downstream side end portion in the puncture direction of the electrode portion 28, so as to prevent the puncture needle 6 to deeply pierce the skin excessively. To be specific, the stopper portion 31 is formed, in the electrode portion 28, such that the furthest downstream tip in the puncture direction of the generally regular pentagon shape when viewed from top is dented toward an upstream side in the puncture direction. That is, the stopper portion 31 is provided, in the electrode portion 28, so as to protrude from both outer sides in the width directions (both widthwise outer sides and an oblique upstream side in the puncture direction) of the puncture needle 6 interposed therebetween. The end edge of the stopper portion 31 at a downstream side in the puncture direction and the distal end 29 of the puncture needle 6 are spaced apart by, for example, 0.3 to 2 mm.

In the circuit board for blood collection 1, a second bending portion 14 is formed at an adjacent portion (that is, corresponding to a boundary between the support portion 5 and the plurality of measurement units 2) where the support portion 5 and the plurality of measurement units 2 are adjacent to each other. The second bending portion 14 is provided so that the support portion 5 is bendable with respect to the plurality of measurement units 2.
The circuit board for blood collection 1 includes, as shown in FIG. 4, a metal substrate 11, an insulating base layer 12 as an insulating layer laminated onto the metal substrate 11, a conductive pattern 7 laminated onto the insulating base layer 12, and an insulating cover layer 13 provided on the insulating base layer 12 so as to cover the conductive pattern 7.

The metal substrate 11 is formed of a metal foil and the like, as shown in FIGs. 1 and 4, and is formed into a shape that corresponds to the outline shape of the circuit board for blood collection 1. That is, the metal substrate 11 is formed as one sheet in one circuit board for blood collection 1.
Examples of the metal material that forms the metal substrate 11 include nickel, chromium, iron, and stainless steel (SUS304, SUS430, and SUS316L). In view of rigidity and retaining the bent form to be mentioned later of the support portion 5 at the time of rolling, stainless steel is preferably used. The thickness of the metal substrate 11 is, for example, 10 to 300 µm, or preferably 20 to 100 µm. When the thickness is below 10 µm, the skin puncturing (described later) may not be performed because of insufficient strength. On the other hand, when the thickness exceeds 300 µm, the puncturing may cause pain and damage the skin excessively, or the first bending portion 45 and/or the second bending portion 14 may not be bent smoothly.

From the metal substrate 11, the upstream-side portion 3, the downstream-side portion 4 (electrode portion 28, and puncture needle 6), and the support portion 5 are formed. Because the puncture needle 6 is formed from the metal substrate 11 made of the above-described metal material, reliable puncturing can be achieved. Furthermore, because the gear 18 of the support portion 5 is formed from the metal substrate 11 made of the above-described metal material, reliable rotation of the circuit board for blood collection 1 can be achieved.

The insulating base layer 12 is formed, in the upstream-side portion 3 and in the downstream-side portion 4, on the surface of the metal substrate 11 corresponding to the upstream-side portion 3 and the downstream-side portion 4; in the support portion 5, on the surface of the metal substrate 11, at a downstream portion in the puncture direction of the metal substrate 11 continuously over the width direction thereof; and in the connector portion 15, so as to correspond to the outline shape of the connector portion 15.
Furthermore, the insulating base layer 12 is formed, as shown in FIG. 2, so as to expose, in the upstream-side portion 3, a downstream side end portion in the puncture direction of the metal substrate 11 when viewed from top. Furthermore, the insulating base layer 12 is formed, as shown in FIG. 3, in the downstream-side portion 4 including the stopper portion 31, so as to bulge from the peripheral end portion of the metal substrate 11, to be more specific, from the outer end portion in the width direction and both end portions in the puncture direction of the metal substrate 11, toward an outer side in the width direction and both puncture directions when viewed from top.

Examples of the insulating material that forms the insulating base layer 12 include synthetic resins such as polyimide resin, polycarbonate resin, polyethylene resin, polyethyleneterephthalate resin, epoxy resin, and fluorocarbon resin. In view of mechanical durability, and chemical resistance, preferably, polyimide resin is used. The thickness of the insulating base layer 12 is, for example, 3 to 50 µm, or preferably 5 to 25 µm. When the thickness is below 3 µm, there may be a case where an insulation defect such as pinholes is caused. On the other hand, when the thickness exceeds 50 µm, cutting and trimming may become difficult.

The conductive pattern 7 is formed, as shown in FIG. 4, on the surface of the insulating base layer 12, and is formed as a wiring circuit pattern including the above-described three electrodes 8, three terminals 9, and three wirings 10.
Examples of the conductive material that forms the conductive pattern 7 include metal materials such as iron, nickel, chromium, copper, gold, silver, platinum, and alloys thereof. The conductive material is selected appropriately in view of adhesiveness to the insulating base layer 12 and the insulating cover layer 13, and easy workability. Two or more conductive materials may be laminated as well. The thickness of the conductive pattern 7 is, for example, 5 to 50 µm, or preferably 10 to 20 µm.

The insulating cover layer 13 is provided on the surface of the insulating base layer 12 so as to cover the wirings 10. The peripheral end portion of the insulating cover layer 13 is, as shown in FIGs. 2 to 4, disposed so as to coincide with the peripheral end portion of the insulating base layer 12 when viewed from top.
The insulating cover layer 13 is formed, as shown in FIG. 4, with electrode-side openings 38 to expose the electrodes 8, and terminal-side openings 39 to expose the terminals 9. To be specific, the electrode-side openings 38 are formed, as shown in FIG. 2, so as to encircle the electrodes 8 and to be slightly larger than the electrodes 8 when viewed from top. The terminal-side openings 39 are formed so as to encircle the terminals 9 and to be slightly larger than the terminals 9 when viewed from top. For the insulating material that forms the insulating cover layer 13, the above-described insulating materials of the insulating base layer 12 are used. The thickness of the insulating cover layer 13 is, for example, 2 to 50 µm.

Then, as shown in FIG. 3, the stopper portion 31 is formed from the bulging portion of the above-described insulating base layer 12 and insulating cover layer 13. The stopper portion 31 can effectively prevent damage to the skin that is brought into contact with the stopper portion 31 when preventing excessive puncturing with the puncture needle 6, because the insulating material that forms the stopper portion 31 is softer than the metal material.
Then, as described above, the connector portion 15 is formed from the insulating base layer 12 and the insulating cover layer 13. Therefore, because the insulating material (synthetic resin) that forms the insulating base layer 12 and the insulating cover layer 13 is softer than the metal material in the circuit board for blood collection 1 formed, the measurement unit 2 can be flexibly connected. Also, at the time of rolling the circuit board for blood collection 1 to be mentioned later, the measurement unit 2 can be flexibly connected while stretching the winding portion of the connector portion 15.

The first bending portion 45 and the second bending portion 14 are formed from the above-described metal substrate 11, insulating base layer 12, and insulating cover layer 13.
Next, with reference to FIGs. 5 and 6, a method for producing the circuit board for blood collection 1 is described.
In this method, first, as shown in FIG. 5(a), a metal foil in which the metal substrate 11 is defined is prepared. For example, an elongated sheet metal foil that is long in the longitudinal direction and that can ensure a large number of the metal substrates 11 is prepared. From such an elongated metal foil, the frame portion 36 and the plurality of circuit board for blood collections 1 are formed by trimming the metal substrates 11 in the subsequent steps.

Next, in this method, as shown in FIG. 5 (b), the insulating base layer 12 is formed on the surface of the metal substrate 11. For the formation of the insulating base layer 12, for example, the following methods are used: a method in which a varnish of a photosensitive synthetic resin is applied on the surface of the metal substrate 11, photoprocessed, and then cured; a method in which a synthetic resin film is laminated onto the surface of the metal substrate 11, an etching resist having the same pattern as that of the insulating base layer 12 is laminated onto the surface of the film, and afterwards, the film exposed from the etching resist is wet-etched; a method in which a synthetic resin film that is punched by a machine in advance is laminated onto the surface of the metal substrate 11; and a method in which a synthetic resin film is laminated onto the surface of the metal substrate 11 first, and then subjected to discharge processing or laser processing. In view of processing accuracy, the method in which a varnish of a photosensitive synthetic resin is applied on the surface of the metal substrate 11, photoprocessed, and then cured is preferably used.

Afterwards, in this method, as shown in FIG. 5 (c), the conductive pattern 7 is formed. For the formation of the conductive pattern 7, a known patterning method for forming printed wirings is used, such as an additive method and a subtractive method. In view of achieving a minute pattern, preferably, the additive method is used. In the additive method, for example, a metal thin film 34 (broken line) is formed on the surface of the insulating base layer 12 by chemical vapor deposition or sputtering, and after a plating resist is formed on the surface of the metal thin film 34, a plating layer 35 is formed on the surface of the metal thin film 34 exposing from the plating resist by electrolytic plating using the metal thin film 34 as a seed film.

The conductive pattern 7 can also be formed only of the metal thin film 34 by chemical vapor deposition or sputtering.
Upon formation of the conductive pattern 7, a different type of metal plating layer may also be formed on the surface of the electrodes 8 and the surface of the terminals 9 by further electrolytic plating or electroless plating, although not shown in the drawings. The thickness of the metal plating layer is preferably 0.05 to 10 µm.

Next, in this method, as shown in FIG. 5(d), the insulating cover layer 13 is formed. For the formation of the insulating cover layer 13, the same method as the method for forming the insulating base layer 12 is used. A preferable method that may be used is the method in which a varnish of a photosensitive synthetic resin is applied on the surface of the insulating base layer 12 so as to cover the conductive pattern 7, photoprocessed, and then cured. When the insulating cover layer 13 is to be formed into a pattern, the electrode-side openings 38 and the terminal-side openings 39 may be formed by forming the insulating cover layer 13 into a pattern having the electrode-side openings 38 and the terminal-side openings 39; and the electrode-side openings 38 and the terminal-side openings 39 may also be formed, for example, by a discharge processing method, and a laser processing method.

Afterwards, as shown in FIG. 6(e), the metal substrate 11 is trimmed to simultaneously form the puncture needle 6; a plurality of measurement units 2 (the upstream-side portion 3 and the downstream-side portion 4 including the puncture needle 6 and the electrode portion 28 (including the stopper portion 31)) that are arranged in parallel in the width direction; the support portion 5 in which the gear 18 and the slit portion 16 are formed; and the first bending portion 45 and the second bending portion 14. By such trimming of the metal substrate 11, the frame portion 36 and the joint portion 37 are simultaneously formed.

For the trimming of the metal substrate 11, for example, discharge processing, laser processing, mechanical punching processing (for example, punching processing), or etching processing is used. In view of easy cleaning after processing, etching processing (wet etching) is preferably used.
By such trimming, with the circuit board for blood collection 1, the plurality of measurement units 2 can be arranged radially by rolling the support portion 5.

In this way, the circuit board for blood collection 1 including the plurality of measurement units 2 and the support portion 5 can be produced in a plural number and in an arranged state within the frame portion 36.
In the obtained circuit board for blood collection 1, as shown in FIG. 6(f), the chemical agent 30 is applied on the electrodes 8: that is, for example, glucose oxidase, or glucose dehydrogenase, as an enzyme, and for example, potassium ferricyanide, ferrocene, or benzoquinone as a mediator, alone or in combination is applied. For the application of the chemical agent 30, for example, an appropriate method such as a dipping method, a spray method, or an inkjet method is used.

Depending on the type of the chemical agent 30, it is also possible to, after the plating layer of a different metal is formed on the surface of the electrodes 8 as described above, further form a coating of a different metal in advance, and provide a predetermined potential difference therebetween. To be specific, for example, after a gold plating layer is formed, silver or silver chloride is applied on the surface of the gold plating layer.
Furthermore, by cutting the joint portion 37 and separating it from the frame portion 36, the circuit board for blood collection 1 is obtained.

FIG. 7 shows schematic perspective views for describing an embodiment of a method for using a circuit board for body fluid collection of the present invention.
Next, with reference to FIG. 7, a method for using the circuit board for blood collection 1 is described.
To use the circuit board for blood collection 1, first, the joint portion 37 is cut as described above and the circuit board for blood collection 1 is separated from the frame portion 36, thereby preparing one circuit board for blood collection 1 as shown in FIG. 7(a). In this method, the circuit board for blood collection I is disposed such that the electrode 8 and the terminal 9 face upward.

Next, in this method, as shown in FIG. 7(b), the support portion 5 is bent upward with respect to the plurality of measurement units 2 at the second bending portion 14.
The angle (bending angle) θ2 between the plurality of measurement units 2 and the support portion 5 is, for example, 45 to 135°, or preferably 60 to 120°. When the bending angle θ2 is outside the above range, there may be a case where reliable puncturing with the measurement unit 2 that performs the measurement cannot be performed, or puncturing with the measurement unit 2 that performs the measurement is inhibited (interrupted) by neighboring measurement units 2.

Next, in this method, as shown in FIG. 7(c), the support portion 5 is rolled, and the plurality of measurement units 2 are arranged radially.
To be specific, the support portion 5 is rolled so that the respective measurement units 2 are extended outwardly in the radial direction with respect to the support portion 5. Furthermore, as shown in FIG. 7(d), by engaging the slit portions 16 at widthwise both end portions of the support portion 5 with each other, the rolling of the support portion 5 is retained. When rolling the support portion 5, the winding portion of the connector portion 15 is stretched, and formed into a generally straight line when viewed from top.

In this fashion, the support portion 5 is rolled, and the circuit board for blood collection 1 in which the plurality of measurement units 2 extend outwardly in the radial direction with respect to the support portion 5 can be obtained.
FIG. 8 shows schematic perspective views of a blood-sugar-level measuring device as an embodiment of the biosensor of the present invention, in which the circuit board for blood collection 1 shown in FIG. 7 is mounted; and FIG. 9 shows side sectional views for describing a method for using the blood-sugar-level measuring device shown in FIG. 8. In FIG. 9, the right side on the plane of the sheet is referred to as "front side", the left side on the plane of the sheet is referred to as "rear side", the upper side on the plane of the sheet is "upper side", the lower side on the plane of the sheet is referred to as "lower side", the front side on the plane of the sheet is referred to as "left side", and the back side on the plane of the sheet is referred to as "right side"; and directions indicated in FIG. 8 are in accordance with the directions in FIG. 9.

Next, with reference to FIGs. 8 and 9, a method for using the circuit board for blood collection 1, and a method for using the blood-sugar-level measuring device 19 in which the circuit board for blood collection 1 is mounted is described.
In FIGs. 8 and 9, the circuit board for blood collection 1 obtained as described above is mounted and used in the blood-sugar-level measuring device 19, for a patient to puncture his/her skin of, for example, finger to collect blood and measure a glucose level in the collected blood as described above.

That is, the blood-sugar-level measuring device 19 includes a casing 41, a blood collection unit 42, a determination unit 43 (omitted in FIG. 8) that measures a glucose level in blood, and a display unit 44.
The casing 41 is prepared to accommodate the members of the blood-sugar-level measuring device 19, and is formed into a box. To be specific, the casing 41 accommodates the blood collection unit 42 and the determination unit 43; and the display unit 44 is provided on the surface of the casing 41. The casing 41 has a front side opening 33, an upper side opening 22, and a bending guide portion 49 formed therewith.

The front side opening 33 is formed in the front wall of the casing 41 so as to extend in the left and right directions to form a generally rectangular shape when viewed from the front, and to expose some (a few units) of the measurement units 2 when the circuit board for blood collection 1 advances forward, as described later.
The upper side opening 22 is formed at a center in the left-right directions of a front side of the upper wall of the casing 41 as a long hole that extends in front and rear directions. To be specific, the upper side opening 22 is formed so that the driving shaft 21, to be described later, is inserted slidably in front and rear directions.

The bending guide portion 49 is provided at a center in the left and right directions of the front wall of the casing 41 and at an upper end edge of the upper side opening 22 of the front wall, and is formed into a generally flat plate. The bending guide portion 49 is provided such that its front end edge is swingable in up and down directions with its back end edge as the supporting point, and is disposed to extend obliquely forward toward a lower side from the front wall of the casing 41 so as to usually block ahead of the upper side opening 22. The bending guide portion 49 closes, when puncturing with the circuit board for blood collection 1, so as to block ahead of the upper side opening 22 (ref: FIG. 9(b)), while when measuring a blood-sugar level, the bending guide portion 49 opens, so as to expose the upper side opening 22 and then to expose the foremost measurement unit 2 therefrom, and so as not to make contact with the foremost measurement unit 2 (ref: FIG. 9(d)).

Furthermore, regarding the bending guide portion 49, when puncturing with the circuit board for blood collection 1, an angle between the direction along the bending guide portion 49 and the front and rear directions when the bending guide portion 49 is viewed from a side is not particularly limited as long as the angle allows a patient easy usage, and is adjusted to an appropriate angle, for example, to be specific, 15 to 60°, or preferably 20 to 45°.
The blood collection unit 42 includes, as shown in FIG. 9, a driving shaft 21, a gear plate 24, a guide portion 23, and the circuit board for blood collection 1.

The driving shaft 21 is disposed so that its axis extends toward up and down directions, and its lower end portion is integrally formed with the gear plate 24.
The gear plate 24 is formed into a generally disc shape, and the driving shaft 21 is integrally inserted to the center of the gear plate 24. On the upper face of the gear plate 24, a plurality of driving grooves 40 extending radially from the center is formed. In this fashion, in the gear plate 24, the gear 18 of the support portion 5 engages with the driving grooves 40. That is, the gear plate 24 is engaged in such a manner that the gear plate 24 is removable along up and down directions from, but not rotatable relative to the gear 18, and is provided so as to allow the circuit board for blood collection 1 to rotate in a circumferential direction with the center of the gear plate 24 (axis of the driving shaft 21) as the center.

The guide portion 23 is provided at a peripheral end of the upper side opening 22 of the upper wall of the casing 41. To be specific, the guide portion 23 is provided so as to guide advancing and retreating of the driving shaft 21 in front and rear directions. The circuit board for blood collection 1 is provided so as to be capable of advancing and retreating in front and rear directions with the driving shaft 21, and rotatable in a circumferential direction with the engagement with the gear plate 24. The circuit board for blood collection 1 is disposed so that the electrodes 8 and the terminals 9 face downward. When the circuit board for blood collection 1 advances, a few measurement unit 2 at the front side expose themselves from the front side opening 33, and among them, the puncture needle 6 of the measurement unit 2 at the foremost side is brought into contact with the bending guide portion 49.

The determination unit 43 is electrically connected to the electrodes 8, and includes a contact portion 26 and a CPU 25.
The contact portion 26 is provided slidably with respect to the terminals 9 so that when the circuit board for blood collection 1 performs a measurement, the contact portion 26 is brought into contact with the terminals 9 (ref: FIGs. 2 and 3) of the measurement unit 2 that performs the measurement. The contact portion 26 is provided so as to be capable of applying a voltage to the electrodes 8 via the terminals 9, as well as capable of detecting a change in a resistance value between the electrodes 8 when the voltage is applied.

The CPU 25 is electrically connected to the contact portion 26 via signal wirings 48, and is also connected to the display unit 44. The CPU 25 calculates a glucose level as a blood-sugar level based on changes in the resistance value between the electrodes 8 detected at the contact portion 26 when the circuit board for blood collection 1 performs a measurement.
The display unit 44 is provided at a rear side of the upper wall of the casing 41; includes, for example, LED; and displays the blood-sugar level measured by the CPU 25.

When using the blood-sugar-level measuring device 19, first, as shown in FIG. 8(a) and FIG. 9(a), the driving shaft 21 is slid toward a rear side so that the blood-sugar-level measuring device 19 is ready, and, for example, a finger of a patient himself/herself is brought to a lower side of the bending guide portion 49. When the driving shaft 21 has been slid to a rear side in advance, there is no need to slide the driving shaft 21.
At this time, in the blood-sugar-level measuring device 19, all of the measurement units 2 in the circuit board for blood collection 1 are accommodated in the casing 41 without being exposed from the front side opening 33.

In this method, next, as shown in FIG. 8(b) and FIG. 9(b), the driving shaft 21 is slid toward a front side to expose the puncture needle 6 from the front side opening 33, and a patient himself/herself punctures his/her finger with the puncture needle 6.
At this time, the circuit board for blood collection 1 is allowed to advance toward a front side to expose a few measurement units 2 out of the measurement units 2 from the front side opening 33, and the foremost measurement unit 2 is brought into contact with the bending guide portion 49, which causes the downstream-side portion 4 to be bent toward an obliquely lower side with respect to the upstream-side portion 3 at the first bending portion 45. Then, the puncture needle 6 of the downstream-side portion 4 that was bent is used to puncture the finger.

Because the bending guide portion 49 is disposed at the above-described predetermined angle when viewed from a side, the bending angle at the first bending portion 45 is, for example, 15 to 60°, or preferably 20 to 45°.
At this time, upon puncturing with the puncture needle 6, when the stopper portion 31 abuts on the skin, further puncturing is restricted. Thus, the puncturing depth of the puncture needle 6 is, for example, 0.5 to 1.5 mm.

Next, in this method, as shown in FIG. 9(c), the driving shaft 21 is slid to a rear side, and the puncture needle 6 is withdrawn from, for example, a finger, causing a trace amount of bleeding from the punctured portion.
At this time, the measurement unit 2 that was bent by the bending guide portion 49 is brought away from the bending guide portion 49, modifying the bending angle. To be specific, the bending angle at the first bending portion 45 is, for example, 15 to 60°, or preferably 15 to 40°.

The bleeding in a trace amount at the punctured portion can be accelerated as necessary by pressing (stressing) in the proximity of the punctured portion.
Next, in this method, as shown in FIG. 9(d), the front end portion of the bending guide portion 49 is swung upward to open, and the driving shaft 21 is slid again toward a front side to expose the electrodes 8 of the foremost measurement unit 2 from the front side opening 33, so as to bring the electrodes 8 closer and in contact with the punctured portion.

Then, the surface of the electrodes 8 is brought into contact with the blood collected by the puncturing with the puncture needle 6, and the blood is reacted with the chemical agent 30. At this time, the contact portion 26 is brought into contact with the terminals 9, and at the same time, a voltage is applied to the electrodes 8 from the contact portion 26 via the terminal 9. Then, a change in the resistance value between the electrodes 8 at the time of the voltage application is detected by the contact portion 26, and based on the change in the resistance value detected by the contact portion 26, the CPU 25 calculates a glucose level as a blood-sugar level. Then, the blood-sugar level measured by the CPU 25 is displayed at the display unit 44.

Afterwards, in this method, although not shown, by rotating the driving shaft 21 and the gear plate 24 in a circumferential direction with the center of the gear plate 24 as the center to rotate the circuit board for blood collection 1, an unused measurement unit 2 that is disposed at an upstream side of and adjacent to the used measurement unit 2 in the rotational direction is disposed at the foremost side. Afterwards, the steps shown in the above-described FIG. 9(a) to FIG. 9(d) are performed several times, thereby measuring the blood-sugar level several times.

Then, with the circuit board for blood collection 1, and the blood-sugar-level measuring device 19 including the circuit board for blood collection 1, by causing bleeding by puncturing with the puncture needle 6, and allowing the electrodes 8 of the measurement unit 2 to be brought into contact with the blood that was caused to bleed, a blood-sugar level can be simply measured by the CPU 25 that is electrically connected with the electrodes 8.
As a result, the circuit board for blood collection 1 and the blood-sugar-level measuring device 19 are capable of simply measuring a blood-sugar level with a simple structure.

Furthermore, with the circuit board for blood collection 1, based on the plurality of measurement units 2 provided in one circuit board for blood collection 1, multiple measurements of a blood-sugar level can be achieved.
Furthermore, with the circuit board for blood collection 1, by rolling the support portion 5 and arranging the plurality of measurement units 2 radially, with rotation of the circuit board for blood collection 1 in a circumferential direction after using one measurement unit 2, the used measurement unit 2 can be changed to an unused measurement unit 2 that is at an upstream side of and adjacent to the used measurement unit 2 in the rotational direction. Therefore, for every measurement in the multiple measurements, the measurement unit 2 can be changed easily.

Furthermore, according to the above-described method for producing the circuit board for blood collection 1, by trimming the metal substrate 11, a plurality of measurement units 2 that are arranged in parallel in the width direction, and the support portion 5 are formed. Therefore, the space for providing the circuit board for blood collection 1 that is arranged in parallel in the frame portion 36 is made compact to achieve space-saving, yields of the circuit board for blood collection 1 can be improved, and an improvement in production efficiency allows a decrease in costs.

Furthermore, according to the above-described method for using the circuit board for blood collection 1, by bending the second bending portion 14 at the boundary between the plurality of measurement units 2 and the support portion 5, and rolling the support portion 5 that was bent, the plurality of measurement units 2 can be arranged radially. Therefore, the plurality of measurement units 2 can be arranged radially by easy procedures.
Furthermore, by bending the second bending portion 14, when using the measurement unit 2, unnecessary contact or damage by the measurement unit 2 that is adjacent to the measurement unit 2 that performs the measurement can be prevented, and only the measurement unit 2 that performs the measurement can be used.

Furthermore, with the circuit board for blood collection 1, the plurality of measurement units 2 arranged at an outside in the radial direction of the rolled support portion 5 allows reliable puncturing and measurement.
Furthermore, with the circuit board for blood collection 1, even though the plurality of measurement units 2 are arranged radially, because the respective measurement units 2 are connected by the connector portion 15, the plurality of measurement units 2 can be reliably supported. Therefore, reliable puncturing and measurement by the measurement unit 2 can be achieved.

Furthermore, with the circuit board for blood collection 1, by engaging the slit portion 16 at the widthwise one end portion with the slit portion 16 at the other end portion of the support portion 5 when rolling the support portion 5, compared with the case where an adhesive is used for adhesion, rolling of the support portion 5 can be reliably and easily retained. Therefore, the plurality of measurement units 2 can be reliably arranged radially.
Although the support portion 5 is bent upward with respect to the plurality of measurement units 2 in which the electrodes 8 and the terminals 9 are exposed upward in the above description of FIG. 7(b) (solid line portion), the present invention is not limited to such an embodiment, and for example, as shown in the broken line in FIG. 7(b), the support portion 5 can be bent downward with respect to the plurality of measurement units 2.

FIGs. 10 and 11 illustrate schematic perspective views for describing other embodiments (embodiment in which the measurement units extend inwardly in the radial direction with respect to the support portion) of the method for using a circuit board for body fluid collection of the present invention: FIG. 10 shows an embodiment in which the support portion is bent upward with respect to the plurality of measurement units, and FIG. 11 shows an embodiment in which the support portion is bent downward with respect to the a plurality of measurement units. In the following figures, the members that are the same as those described above are designated by the same reference numerals, and descriptions thereof are omitted.

In the above descriptions of FIG. 7(c) and FIG. 7(d), the support portion 5 is rolled so that the respective measurement units 2 extend outwardly in the radial direction with respect to the support portion 5, the direction of the extension of the respective measurement units 2 with respect to the support portion 5 is not limited thereto, and for example, as shown in FIGs. 10 and 11, the support portion 5 may be rolled so that the respective measurement units 2 extend inwardly in the radial direction with respect to the support portion 5.

By rolling the support portion 5 so that the measurement units 2 extend inwardly in the radial direction with respect to the support portion 5, with the plurality of measurement units 2, reliable puncturing and measurement can be achieved at the inside of the rolled support portion 5 in the radial direction.
FIGs. 12 and 13 illustrate enlarged plan views of measurement units of a circuit board for blood collection as other embodiments of a circuit board for body fluid collection of the present invention: FIG. 12 shows an embodiment in which the connector portion is generally W-shaped when viewed from top, and FIG. 13 shows an embodiment in which the connector portion is a straight line when viewed from top.

Although the connector portion 15 is formed into a generally S-shape when viewed from top in the description above, the form is not particularly limited, and the connector portion 15 can be formed into an appropriate shape. For example, as shown in FIG. 12, the connector portion 15 may be formed into a generally W-shape when viewed from top, or as shown in FIG. 13, the connector portion 15 may be formed into a straight line along the width direction when viewed from top.
When the support portion 5 is provided so that the measurement units 2 extend outwardly in the radial direction with respect to the support portion 5, preferably, the connector portion 15 is formed into a generally S-shape when viewed from top (FIGs. 2 and 3) or a generally W-shape when viewed from top (FIG. 12). In this way, a predetermined length is ensured between the measurement units 2 before the rolling, and therefore the measurement units 2 are reliably connected while the connector portion 15 is stretched when the support portion 5 is rolled. The length of the connector portion 15 between the measurement units 2 before rolling is, for example, 0.5 mm or more, or preferably 2 mm or more, and generally 10 mm or less.

As shown in FIG. 13, when the connector portion 15 is to be formed into a straight line when viewed from top, preferably, the support portion 5 is rolled so that the measurement units 2 extend inwardly in the radial direction with respect to the support portion 5. However, when the support portion 5 is rolled so that the measurement units 2 extend outwardly in the radial direction with respect to the support portion 5, the connector portion 15 may be cut.
Although the connector portion 15 is formed from the insulating base layer 12 and the insulating cover layer 13 in the above description, the layer structure of the connector portion 15 is not limited thereto. For example, although not shown, the connector portion 15 may also be formed from only one of the insulating base layer 12 and the insulating cover layer 13.

Although 32 units of the measurement unit 2 are provided in the circuit board for blood collection 1 in the description above with reference to FIG. 1, the number is not particularly limited, and is selected appropriately according to the size of the casing 41 and the like. For example, 10 or more, or preferably 20 or more, and generally 70 or less measurement units 2 may be provided. The size of the circuit board for blood collection 1 is appropriately selected according to the size of the casing 41 or the number of the measurement unit 2 in the description above with reference to FIG. 1. For example, without limitation, the length of the circuit board for blood collection 1 in the puncture direction (the length between the end portion of the support portion 5 in the upstream side in the puncture direction and the distal end 29 of the puncture needle 6) is 3 to 50 mm, or preferably 5 to 15 mm. When the length of the circuit board for blood collection 1 in the puncture direction is below the above-described range, the electrode 8 becomes excessively small, and formation of the electrode 8 and the application of the chemical agent 30 may become difficult. On the other hand, when the length in the puncture direction of the circuit board for blood collection 1 exceeds the above-described range, the yields of the circuit board for blood collection 1 may decrease and cause an increase in costs.

The length in the width direction (or the length between widthwise both end portions of the support portion 5) of the circuit board for blood collection 1 is, for example, 50 to 300 mm, or preferably 80 to 150 mm. When the length in the width direction of the circuit board for blood collection 1 is below the above-described range, the number of the measurement unit 2 may become excessively small. On the other hand, when the length in the width direction of the circuit board for blood collection 1 exceeds the above-described range, the yields of the circuit board for blood collection 1 may decrease and cause an increase in costs.

In the description above with reference to FIGs. 1 and 8, the gear 18 is provided at about the entire face of the end face at an upstream side in the puncture direction of the support portion 5. However, for example, although not shown, the gear 18 may be provided at only a portion of the end face in the upstream side in the puncture direction of the support portion 5, and the rest of the face may be formed into a flat face (without projections and recesses). In such a case, the driving grooves 40 (ref: FIG. 8) of the gear plate 24 are formed into the shape that mate with the above-described gear 18.

Furthermore, the gear 18 may be formed so as to engage, like a key, with the driving grooves 40 of the gear plate 24 formed into a key.
In the description above, the circuit board for blood collection 1 and the blood-sugar-level measuring device 19 including the circuit board for blood collection 1 are given as examples of the circuit board for body fluid collection and the biosensor including the circuit board for body fluid collection of the present invention. That is, description is given using blood as the body fluid collected by puncturing with the puncture needle of the circuit board for body fluid collection.

However, the body fluid is not particularly limited as long as it is a liquid in a living body, and examples thereof include extracellular fluid and intracellular fluid. Examples of the extracellular fluid include, other than blood mentioned above, a blood plasma; an intercellular fluid; a lymph fluid; moistures in dense connective tissue, bone, and cartilage; and a transcellular fluid. A measurement on a specific component of the above-described body fluid can be performed with the circuit board for body fluid collection and the biosensor including the circuit board for body fluid collection.

### EXAMPLES

### EXAMPLE 1

### (production of Circuit Board for Blood Collection Shown in FIG. 1)

First, an elongated sheet metal foil made of SUS 304, in which a metal substrate was defined, and having a thickness of 50 µm and a width of 300 mm was prepared (ref: FIG. 5(a)).
Then, on the surface of the metal substrate, a varnish of a photosensitive polyimide resin precursor (photosensitive polyamic acid resin) was applied, and dried by heating to form a coating. The coating was then exposed to light, and developed to be formed into a pattern. Thereafter, the coating was heated in a nitrogen atmosphere to 400°C, to form an insulating base layer having a thickness of 10 µm in the abovementioned pattern (ref: FIG. 5 (b)).

Then, on the surface of the insulating base layer, metal thin films formed of a chromium thin film and a copper thin film were formed sequentially by sputtering. Subsequently, a dry film resist was laminated on the surface of the metal thin film, exposed to light, and developed to form a plating resist in a pattern. Then, a plating layer formed of copper was formed on the surface of the metal thin film exposed from the plating resist using the metal thin film as a seed film by electrolytic copper plating, thereby forming a conductive pattern including electrodes, terminals, and wirings (ref: FIG. 5 (c)). Afterwards, the plating resist and the portion of the metal thin film where the plating resist was formed were removed by etching.

The conductive pattern had a thickness of 12 µm, the two electrodes (8a) had a diameter of 0.3 mm, and the long side of the one electrode (8b) had a length of 1.0 mm, and the short side of the one electrode (8b) had a length of 0.6 mm. The length of a side of the two terminals (9a) was 3 mm, and the length of a side of the one terminal (9b) was 1 mm. The width of the wirings was 100 µm; the length of the wirings that connect the two electrodes 8a and the two terminals 9a was 3 mm; and the length of the wiring that connects the one electrode 8b and the one terminal 9b was 7 mm.

Afterwards, a varnish of a photosensitive polyimide resin precursor (photosensitive polyamic acid resin) was applied on the surface of the insulating base layer so as to cover the conductive pattern, and dried by heating to form a coating. The coating was then exposed to light, and developed to be formed into a pattern. Thereafter, the coating was heated under a nitrogen atmosphere at 400°C, thereby forming an insulating cover layer having a thickness of 5 µm (ref: FIG. 5(d)). The insulating cover layer was formed such that by forming the electrode-side openings and terminal-side openings, the electrodes and the terminals were exposed but the wirings were covered.

Thereafter, an electrolytic nickel plating layer (thickness 0.5 µm), and an electrolytic gold plating layer (thickness 2.5 µm) were sequentially formed on the surface of the electrodes and the terminals.
Then, a dry film resist was laminated on the surface of the metal substrate, exposed to light, and developed to form an etching resist in a pattern. Then, the metal substrate exposed from the etching resist was wet-etched using ferric chloride as the etching solution, and trimmed into the above-described pattern, i.e., a plurality of measurement units arranged in parallel in the width direction including a puncture needle; a support portion in which the gear and the slit portion are formed; and the first bending portion and the second bending portion (ref: FIG. 6(e)). By such trimming of the metal substrate, the frame portion and the joint portion were simultaneously formed.

The length from the distal end of the puncture needle to the one electrode (8b)(the electrode nearest from the distal end) was 1.8 mm; the angle at the distal end of the puncture needle was 20°; the width of the bulging portion of the stopper portion was 0.4 mm; the end edge at the downstream side in the puncture direction of the stopper portion and the distal end of the puncture needle were spaced apart by 1.4 mm.
The circuit board for blood collection was thus obtained. The circuit board for blood collection had a length in the width direction of 2.7 mm, and had a length in the puncture (longitudinal) direction of 10 mm.

Afterwards, in the obtained circuit board for blood collection, a chemical agent containing glucose oxidase and a potassium ferricyanide solution was applied on the electrode in respective measurement units by inkjet (ref: FIG. 6 (f)).
Thereafter, by cutting the joint portion, the circuit board for blood collection was detached from the frame portion, and the circuit board for blood collection was disposed so that the electrodes and the terminals are facing upward (ref: FIG. 7(a)). Then, the support portion was bent upward at 90° (ref: FIG. 7(b)), at the second bending portion with respect to the plurality of measurement units.

Then, the support portion was rolled so that the respective measurement units extend outwardly in the radial direction with respect to the support portion, and the slit portions at the widthwise both end portions of the support portion was engaged with each other, so as to retain the rolling of the support portion. The plurality of measurement units were thus arranged radially (ref: FIG. 7(c) and FIG. 7(d)).

### (Production of the blood-sugar-level measuring device shown in FIGs. 8 and 9)

The rolled circuit board for blood collection was mounted along with the driving shaft, the gear plate, and the guide, in the casing provided with a display unit (ref: FIGs. 8 and 9).

To mount the circuit board for blood collection, the driving grooves of the gear plate integrally formed with the driving shaft was engaged with the gear, and the driving shaft was inserted in the guide portion slidably.

### (Blood-Sugar Level Measurement with Blood-Sugar-Level Measuring Device)

First, the above-described blood-sugar-level measuring device was prepared, and then the finger of the patient himself/herself was brought to a lower side of the bending guide portion (ref: FIG. 8(a) and FIG. 9(a)).

Next, the driving shaft was slid to a front side, to expose the puncture needle from the front side opening, and the patient himself/herself punctured his/her finger with the puncture needle (ref: FIG. 8(b) and FIG. 9(b)). At this time, by exposing a few measurement units out of the measurement units from the front side opening and bringing the foremost measurement unit into contact with the bending guide portion, the upstream-side portion was bent at 40° at the bending portion with respect to the upstream-side portion. Then, the finger was punctured with the puncture needle of the upstream-side portion that was bent.

Next, the driving shaft was slid to a rear side, and the puncture needle was withdrawn from the finger, thus causing a trace amount of bleeding from the punctured portion (ref: FIG. 9(c)). The measurement unit that was bent at the bending portion was thus brought away from the bending guide portion, forming a bending angle of 35° at the bending portion, and modifying the bending angle.
Then, the bending guide portion was opened, and the driving shaft was slid toward a front side again to expose the electrode of the foremost measurement unit from the front side opening, so as to bring the electrode closer and into contact with the punctured portion (ref: FIG. 9(d)).

Then, glucose was oxidized by the blood, and ferricyanide ions reacted. At the same time, a voltage was applied from the contact portion to the electrodes via the terminals. Then, a change in the resistance value between the electrodes at the time of the voltage application was detected by the contact portion, and the CPU calculated a glucose level as a blood-sugar level based on the change in the resistance value. Then, the blood-sugar level measured by the CPU was displayed at the display unit.
Afterwards, in this method, the driving shaft was rotated, and the gear plate was rotated with the center of the gear plate as the center in a circumferential direction, to rotate the circuit board for blood collection, thereby disposing an unused measurement unit disposed at an upstream side of and adjacent to the used measurement unit in the rotational direction at the foremost position. Thereafter, the above-described steps were performed, thus measuring a blood-sugar level a plurality of times (32 times in total).

While the illustrative embodiments of the present invention are provided in the above description, such is for illustrative purpose only and it is not to be construed as limiting the scope of the present invention. Modification and variation of the present invention that will be obvious to those skilled in the art is to be covered by the following claims.

### Industrial Applicability

A circuit board for body fluid collection; a method for producing the same; a method for using the same; and a biosensor including the same of the present invention are suitably used, for example, in the field where a blood-sugar level in blood is measured.

## Claims

1. A circuit board for body fluid collection comprising:
a plurality of measurement units including a puncture needle and an electrode for making contact with a body fluid collected by puncturing with the puncture needle, the plurality of measurement units being arranged in parallel in a predetermined direction, and
a support portion extending along the parallel arrangement direction and supporting the plurality of measurement units,
wherein the support portion can be rolled so that the plurality of measurement units are arranged radially.

2. The circuit board for body fluid collection according to Claim 1, wherein the plurality of measurement units extend outwardly with respect to the support portion when the plurality of measurement units are arranged radially.

3. The circuit board for body fluid collection according to Claim 1, wherein the plurality of measurement units extend inwardly with respect to the support portion when the plurality of measurement units are arranged radially.

4. The circuit board for body fluid collection according to Claim 1, further comprising a reinforcing portion that is disposed in a spaced apart relationship to the support portion, and connects, between the plurality of measurement units, the plurality of measurement units that are adjacent to each other.

5. The circuit board for body fluid collection according to Claim 1, further comprising engage portions at one end portion and at the other end portion of the support portion in the parallel arrangement direction for retaining the rolling of the support portion.

6. A circuit board for body fluid collection comprising:
a plurality of measurement units including a puncture needle and an electrode for making contact with a body fluid collected by puncturing with the puncture needle, the plurality of measurement units being arranged in parallel in a predetermined direction, and
a support portion extending along the parallel arrangement direction and supporting the plurality of measurement units,
wherein the plurality of measurement units are arranged radially by bending a boundary between the plurality of measurement units and the support portion, and rolling the support portion.

7. The circuit board for body fluid collection according to Claim 6, wherein the plurality of measurement units extend outwardly with respect to the support portion.

8. The circuit board for body fluid collection according to Claim 6, wherein the plurality of measurement units extend inwardly with respect to the support portion.

9. A biosensor comprising:
the circuit board for body fluid collection according to Claim 1, and
a determination unit that is electrically connected to the electrodes and performs a measurement on a component of the body fluid.

10. A biosensor comprising:
the circuit board for body fluid collection according to Claim 6, and
a determination unit that is electrically connected to the electrode and performs a measurement on a component of the body fluid.

11. A method for producing a circuit board for body fluid collection, the method including the steps of:
preparing a metal substrate,
forming an insulating layer on the metal substrate,
forming an electrode for making contact with a body fluid on the insulating layer, and
forming, by trimming the metal substrate, a plurality of measurement units including a puncture needle for collecting a body fluid by puncturing and the electrode, the plurality of measurement units being arranged in parallel in a predetermined direction, and a support portion extending along the parallel arrangement direction and supporting the plurality of measurement units,
wherein in the step of forming the plurality of measurement units and the support portion, the metal substrate is trimmed so that by rolling the support portion, the plurality of measurement units can be arranged radially.

12. A method for using a circuit board for body fluid collection, in which the circuit board for body fluid collection produced by the method for producing a circuit board for body fluid collection according to Claim 11 is used, the method comprising the steps of:
bending a boundary between the plurality of measurement units and the support portion, and
rolling the support portion, thereby arranging the plurality of measurement units radially.
